# EUROPEAN PATENT SPECIFICATION

(11) **EP 3 208 345 B1**
(45) Date of publication and mention of the grant of the patent: **04.12.2019**
(21) Application number: 14904140.2
(22) Date of filing: 14.10.2014
(51) Int. Cl.: C12Q 1/6806, C12Q 1/6813, C12Q 1/686, C12N 9/12, C07H 21/00

(54) **ONE-STOP TREATMENT METHOD FOR BREAKING NUCLEIC ACID BY MEANS OF TRANSPOSASE, AND REAGENT**
BEHANDLUNGSVERFAHREN IN EINEM SCHRITT ZUM BRECHEN VON NUKLEINSÄURE MITTELS TRANSPOSASE SOWIE REAGENZ
PROCÉDÉ DE TRAITEMENT D'ARRÊT POUR ROMPRE UN ACIDE NUCLÉIQUE AU MOYEN D'UNE TRANSPOSASE, ET RÉACTIF

(43) Date of publication of application: 23.08.2017
(73) Proprietor: MGI Tech Co., Ltd., Yantian District Shenzhen Guangdong 518083 (CN)
(72) Inventor: GENG, Chunyu, Shenzhen Guangdong 518083 (CN); GUO, Rongrong, Shenzhen Guangdong 518083 (CN); CHEN, Ruoying, Shenzhen Guangdong 518083 (CN); ZHANG, Yingxin, Mountain View, California 94040 (US); ALEXEEV, Andrei, Woodland, California 95776 (US); JIANG, Hui, Shenzhen Guangdong 518083 (CN); ZHANG, Wenwei, Shenzhen Guangdong 518083 (CN)
(74) Representative: Hamer, Christopher K.
(86) International application number: PCT/CN2014/088541
(87) International publication number: WO 2016/058126

(56) References cited:
- EP-A1- 2 690 180
- EP-A1- 3 192 900
- WO-A1-2010/048605
- WO-A1-2014/142850
- CN-A- 103 710 323
- CN-A- 103 938 277
- WANG QI ET AL: "Tagmentation-based whole-genome bisulfite sequencing", NATURE PROTOCOLS, NATURE PUBLISHING GROUP, GB, vol. 8, no. 10, 1 October 2013 (2013-10-01), pages 2022-2032, XP009178706, ISSN: 1750-2799, DOI: 10.1038/NPROT.2013.118

## Description

### TECHNICAL FIELD

The present invention relates to the field of molecular biology, and more particularly to one-stop treatment methods and reagents for breaking a nucleic acid by means of a transposase.

### BACKGROUND OF THE INVENTION

Since the pyrophosphate sequencing method invented by Roche, which has opened up the next generation of sequencing, until now, the next generation of sequencing has undergone a period of rapid development. However, with the development of high-throughput sequencing, the sample preparation with high-throughput and low-cost has become a key consideration in the field of sequencing. Sample processing methods and automation devices of various principles have been developed, including: samples fragmentation, terminal treatment of nucleic acid molecules and adapters ligation and the generation of final libraries.

The methods of samples fragmentation mainly include physical methods (such as ultrasound shear) and enzymatic methods (i.e., treatment of non-specific endonuclease). Wherein the physical methods are dominated by Covaris based on patented Adaptive Focused Acoustic (AFA) technology. Under an isothermal condition, the acoustic energy with a wavelength of 1 mm is focused on a sample by a spherical solid state ultrasonic sensor with >400kHz, using geometric focusing acoustic energy. This method ensures the integrity of nucleic acid samples, and a high recovery rate can be achieved. Covaris's instruments include an economical M-series, a single-tube full-power S-series and higher-throughput E- and L-series. The randomization of fragments based on physical methods is good, but the physical methods depend on a large number of Covaris interrupters, and require subsequent separate terminal treatment, adapter ligation and PCR, and various purification operations. Wherein the enzymatic methods include the NEB Next dsDNA Fragmentase from NEB company. The reagent first cleaves the double stranded DNA to produce a random cleavage site, and then clears the complementary DNA strand by identifying the cleavage site through another enzyme to achieve the purpose of interruption. This reagent can be used for genomic DNA, whole genome amplification products and PCR products, and randomness is also good, but some artificial short fragments insertion and deletion will be generated. And also inevitably need to carry out subsequent separate terminal treatment, adapter ligation and PCR, and various purification operations. In addition, the transposase disrupting kit led by Nextera kit of Epicentra company (acquired by Illumina) has been used to complete the DNA fragmentation and the adapters ligation simultaneously using the transposase, thereby reducing the time of sample processing.

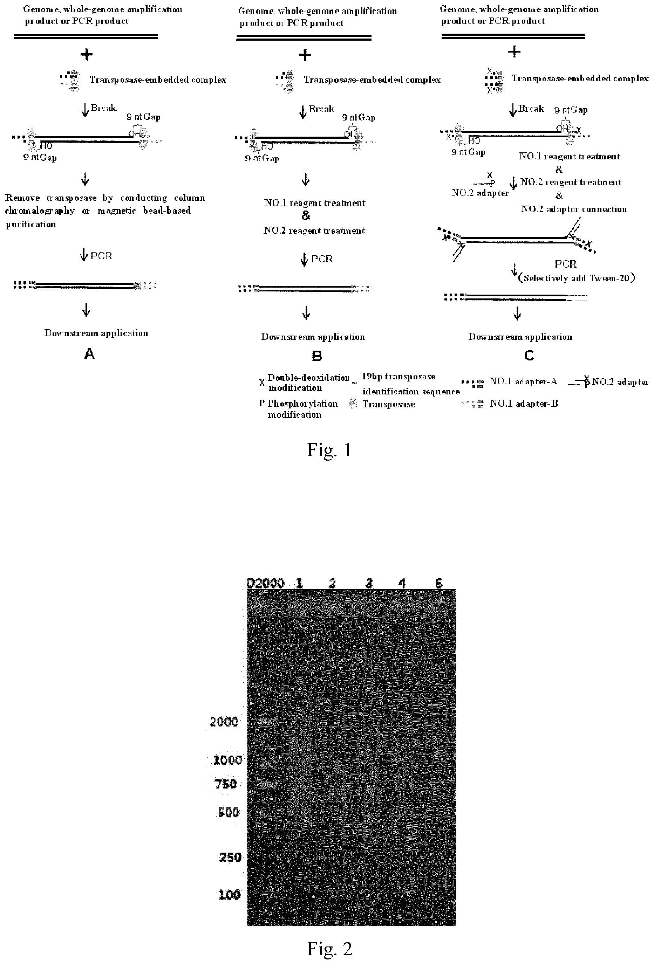

From the simplicity of the various operations, the method of interruption by transposase is far superior to other methods in terms of flux and ease of operation, but this interruption has its own shortcomings: subsequent enzymatic reactions will be inhibited by the transposase embedded in the target sequence, although relevant transposase kit manufacturers provide some reagents for low starting amounts sample (e.g., 5ng of the starting amount of genomic DNA) transposase treatment to achieve free-purification after interruption. However, for a method with 50ng starting amount which requires to increase the amount of transposase, it is necessary to remove the transposase by means of column or magnetic beads purification, which undoubtedly increases the cost and process of experimental procedures (Figure 1A).

### SUMMARY OF THE INVENTION

The present invention provides a one-stop treatment method and reagent for breaking a nucleic acid by means of a transposase, which are capable of achieving a one-stop treatment of from a nucleic acid interruption by transposase to a downstream PCR amplification reaction, and column or magnetic beads purification is not required, thus simplifying the experimental operating procedures and reducing experimental costs.

According to a first aspect of the present invention, a one-stop treatment method, as defined in the appended claims, for breaking a nucleic acid by means of a transposase is provided, comprising the following steps:
randomly interrupting a nucleic acid using a transposase-embedded complex, wherein the transposase-embedded complex comprises a transposase and a first adapter comprising a transposase identification sequence;
adding a first reagent for treatment, so as to break the adsorption effect of the transposase and the target sequence of the nucleic acid;
adding a second reagent for treatment, so as to weaken the influence of the first reagent on the subsequent enzymatic reactions; and
performing a PCR reaction by using a product generated after the second reagent treatment as a template component, so as to obtain a PCR product with an adapter at each end of the interrupted nucleic acid fragment.

In the method of the present invention, the reaction product of the nucleic acid interruption by the transposase is treated with the first reagent to break the adsorption effect of the transposase and the target sequence of the nucleic acids, replacing the conventional column or magnetic beads purification which is complicated process and costly; followed by treatment with a second reagent to weaken the influence of the first reagent on the subsequent enzymatic reaction, ensuring that downstream PCR amplification proceeds smoothly.

As a preferred embodiment of the present invention, the first reagent comprises a sodium dodecyl sulfate (SDS) solution and a NT buffer. These solutions allow the transposase to degrade or denature and escape from the target sequence of the nucleic acids. It is to be noted that the first reagent may comprise solutions, such as a protease solution and the SDS solution, the SDS solution and the NT buffer, the protease solution and the NT buffer, the protease solution, the SDS solution and the NT buffer, wherein the NT buffer can be the NT buffer in S5 series of Truprep kit.

As a preferred embodiment of the present invention, ethylenediaminetetraacetic acid (EDTA) is further added for treatment after the treatment with the first reagent, if the first reagent comprises a protease solution. EDTA inhibits protease activity and thus prevents proteases from degrading enzymes in subsequent PCR reactions.

As an embodiment of the present invention, the second reagent comprises Triton-X100 solution. Triton-X100, whose chemical name octylphenyl polyoxyethylene ether, as a nonionic surfactant, in the role of the present invention is to weaken the influence of the first reagent on the subsequent enzymatic reactions, and the second reagent further comprises a Tween-20. The addition of Tween-20 could further weaken the influence of SDS on the subsequent enzymatic reaction and enhance the PCR effect. It should be noted that Tween-20 may be used as a component of the second reagent in the form of a mixture with Triton-XlOO; it may also be provided separately in the form of separation from Triton-X100, in which case the second reagent refers to the Triton-X100 solution and the Tween-20 solution. It is to be understood that the first reagent and the second reagent in the present invention are not intended to be limited to a single object or a combination of a plurality of objects. Also, in the present invention, concepts such as "first" and "second", which are used in any case, should not be construed as having the meaning of order or technique, instead their role in the present invention is to distinguish themselves from other objects.

As a preferred embodiment of the present invention, after adding the second reagent for treatment and before performing the PCR reaction, a second adapter is ligated at a gap by a ligase, wherein the gap is a 9bp base deletion formed after both ends of the interrupted nucleic acid each ligated to the first adapter. The incorporation of the second adapter makes it possible to obtain a PCR product with different adapter-sequences at both ends, when performing a PCR reaction by using a primer that specifically targets the first adapter and a primer that specifically targets the second adapter, respectively. Thereby the application of the interrupted nucleic acids is not limited by the effect of the presence of transposase identification sequences at both ends. The sequence of the second adapter is not limited and can be any sequence.

As a preferred embodiment of the present invention, the 3' end of the second adapter is a dideoxynucleotide to prevent the second adapter from ligating to the first adapter.

According to a second aspect of the present invention, a one-stop treatment reagent for breaking a nucleic acid by means of a transposase is provided, the reagent comprises the following components:
a first reagent comprising an SDS solution and a NT buffer to break the adsorption effect of the transposase and the target sequence of the nucleic acids; and
a second reagent comprising a Triton-X100 solution to weaken the influence of the first reagent on the subsequent enzymatic reactions.

It is to be noted that the first reagent may also comprise a protease solution, a SDS solution and a NT buffer, wherein more of the above solutions may be two or three above solutions, such as the protease solution and the SDS solution, the SDS solution and the NT buffer, the protease solution and the NT buffer, the protease solution, the SDS solution and the NT buffer, wherein the NT buffer can be the NT buffer in S5 series of Truprep kit.

As a preferred embodiment of the present invention, the first reagent further comprises an additional reagent containing EDTA if the first reagent comprises a protease solution. EDTA inhibits protease activity and prevents proteases from degrading enzymes in subsequent PCR reactions. The additional reagents containing EDTA are provided as part of the first reagent separately from the protease solution.

As an embodiment of the present invention, the second reagent further comprises a Tween-20 solution. Tween-20 may be provided as a component of the second reagent in the form of a mixture with Triton-X100, or may be provided separately from the Triton-X100.

As a preferred embodiment of the present invention, the reagent further comprises a transposase and a first adapter comprising a transposase identification sequence for forming a transposase-embedded complex to randomly interrupte the nucleic acids.

As a preferred embodiment of the present invention, the reagent further comprises PCR components for carrying out a PCR reaction using the product generated by the treatment of the second reagent as a template component. Wherein, the PCR components are well known to include DNA polymerase, PCR buffer, dNTPs, Mg²⁺ solution and primer, etc.

As a preferred embodiment of the present invention, the reagent further comprises a second adapter component for ligation into the gap formed by ligating the first adapter to the interrupted nucleic acid at both ends.

As a preferred embodiment of the present invention, the reagent further comprises a ligase component for ligating a second adapter to the gap formed by ligating the first adapter to the interrupted nucleic acid at both ends.

In the present invention, the nucleic acids to be interrupted may be a genomic DNA, a whole genome amplification product or a PCR product, which may be DNA or cDNA, and may be not limited to the source of the nucleic acids and may be nucleic acid samples derived from an animal, a plant or a microorganism.

In the present invention, the working concentration of the first reagent and the second reagent can be determined empirically by those skilled in the art. In general, in the first reagent, the working concentration of the protease is preferably from 50 to 5000 mAU / mL, more preferably from 75 to 3750 mAU / mL, most preferably 1500 mAU / mL; the working concentration of EDTA is preferably from 1 to 50mmol / L, more preferably 14 mmol / L; the working concentration of SDS is preferably from 0.01% to 1.5% (by volume), more preferably 1% (by volume); the final concentration of NT buffer can be used according to 1×. In the second reagent, the working concentration of Triton-X100 is preferably from 0.1% to 2% (by volume), more preferably 1% (by volume); the working concentration of Tween-20 is preferably from 0.1% to 2% (by volume), more preferably 0.5% (by volume).

The method of the present invention utilizes the first reagent and the second reagent to treat the product of nucleic acids interrupted by the transposase, instead of the traditional column or magnetic beads purification, to achieve the one-stop treatment from the transposase interruption of the nucleic acids to the downstream PCR amplification. The whole process is carried out in a single tube, simplifying the experimental operating flow and reducing the cost of the experiment, shortening the processing cycle, making high-throughput sample processing possible.

### BRIEF DESCRIPTION OF THE DRAWINGS

Figure 1 is an operation flow chart (A) from the interruption by conventional transposase kits to PCR reaction, an operation flow chart (B) of the present invention of one-stop reaction with free-purification, and an operation flow chart (C) of the present invention of one-stop reaction with free-purification, which is optimized according to the introduction of a NO. 1 adapter.
Figure 2 is a result of an electrophoresis of the PCR product of Example 1 of the present invention, in which the PCR product is a product from a NO. 1 adapter double-adapters transposase complex, wherein D2000 is a DNA ladder lane; lane 1 is the purification result by 1×PBI, 1.3×Ampure XP beads; lane 2 is the treatment result by 2 µL Protease + 14 mM EDTA + 1% Triton-X100; lane 3 is the treatment result by 1% SDS + 1% Triton-X100 + 0.5% Tween-20 treatment; lane 4 is the treatment result by NT Buffer + 1% Triton-X100; lane 5 is the treatment result by 2 µL protease + 1% Triton-X100.

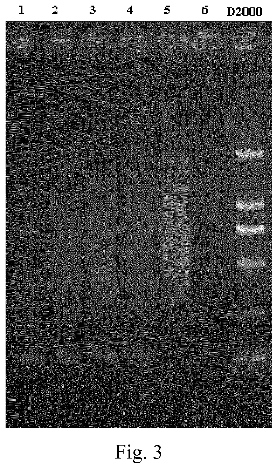

Figure 3 is a result of an electrophoresis of the PCR product of Example 2 of the present invention, in which the PCR product is a product from a NO. 1 adapter single-adapter transposase complex, wherein D2000 is a DNA ladder lane; lane 1 is the treatment result by 2 µL protease + 1% Triton-X100 treatment; lane 2 is the treatment result by NT Buffer + 1% Triton-X100; lane 3 is the treatment result by 1% SDS + 1% Triton-X100 + 0.5% Tween-20; lane 4 is the treatment result by 2 µL protease + 14 mM EDTA + 1% Triton-X100; lane 5 is the treatment result by 1×PBI, 1.3×Ampure XP beads +1%Triton-X100; lane 6 is the result of negative control (no template).

### DETAILED DESCRIPTION OF THE INVENTION

The invention will now be described in further detail by way of specific examples. Unless otherwise specified, the techniques used in the examples below are conventional techniques known to those skilled in the art; the instruments and reagents used are accessable to those skilled in the art through public approaches such as commercial approaches and so on.

Referring to Figure 1B, an operation flow chart of the present invention of one-stop reaction with free-purification mainly comprises: (1) a NO. 1 adapter (elsewhere referred to in the present invention as "first adapter") where a specific modification sequence (including a transposase identification sequence) is embedded by a transposase is used to randomly interrupte nucleic acid sequences, such as genomic sequences, whole genome amplification sequences, or PCR product sequences, etc; (2) breaking the adsorption of the transposase and the target sequence to achieve free-purification by the treatment with protease or other reagent NO. 1 (elsewhere referred to in the present invention as the "first reagent") with specific proportions; (3) adding a reagent No. 2 (elsewhere referred to as the "second reagent" in the present invention) to the product of the previous reaction to weaken the influence of the reagent NO. 1 on subsequent enzymatic reactions; (4) performing a PCR reaction directly without purification mediating through specific primers, wherein the primers were targeted to the NO. 1 adapter, which comprises two adapter portions, each of which comprises a common transposase identification sequence and other sequences different from each other, to obtain a PCR product in which both ends of the target sequence are ligated respectively to a completely different sequence except the transposase identification sequence, thus the product can be used in subsequent molecular biology experiments.

Referring to Figure 1C, an operation flow chart of the present invention of one-stop reaction with free-purification, optimized according to the introduction of a NO. 1 adapter, mainly comprises: (1) a NO. 1 adapter where a specific modification sequence is embedded by a transposase is used to randomly interrupte nucleic acid sequences, such as genomic sequences, whole genome amplification sequences, or PCR product sequences, etc; (2) breaking the adsorption of the transposase and the target sequence to achieve free-purification by the treatment with protease or other reagent NO. 1 with specific proportions; (3) adding a reagent NO. 2 to weaken the influence of the reagent NO. 1 on subsequent enzymatic reactions; (4) ligating a NO. 2 adapter (elsewhere in the present invention are also referred to as a "second adapter" or a "gap adapter") into a 9nt gap, and thus achieving the introduction of the NO. 2 adapter, and changing the adapter base sequence adjacent to the fragmented target sequence, so that the sequences on both sides of the target sequence are completely different, and one of them remains the NO. 1 adapter sequence containing the transposase identification sequence, and the other is completely arbitrarily designed NO. 2 adapter sequence; (5) performing a PCR reaction directly mediating through specific primers, wherein the primers were targeted respectively to the NO. 1 adapter and the NO. 2 adapter, to obtain a PCR product in which both ends of the target sequence are ligated respectively to a completely different sequence, thus the product can be used in subsequent molecular biology experiments.

In the transposase embedding stage, the present invention selectes two embedding modes: the first one is the transposase embedding the NO. 1 adapter in a double-adapters form to generate a transposase-embedded complex (Fig. 1B), both of the two adapters contain a common specific 19 bp transposase identification sequence, but also contain other sequences different from each other. The other one is the transposase embedding the NO. 1 adapter in a single-adapter form to generate a complex (Fig. 1C). Through a special modification of the embedding adapter, the sequence of the NO. 1 adapter embedded contains a portion containing a specific 19 bp transposase identification sequence. At the same time, in order to avoid the inter-ligation between the NO. 1 adapter unembedded and the NO. 2 adapter and to avoid affecting PCR, a 3' end dideoxy modification is carried out in the present invention, i.e., the 3' end is a dideoxynucleotide.

In the present invention, a transposase kit of domestic production (S50 series of Truprep kit of Nanjing Nuoweizan Ltd.) was used to carry out the following experiment. The kit containes two doses respectively for 5ng genomic DNA and 50ng genomic DNA. In this embodiment, the dose for 50ng genomic DNA was used to carry out an experiment.

### Example 1

In this example, 50ng of high quality genomic DNA was first interrupted by an embedded transposase complex, followed by treating with protease, SDS, NT or a composition of protease and EDTA to remove the transposase protein bound to DNA; and then directly amplified using PCR primers, with a certain concentration of TritonX-100 is added into the PCR reaction system.
1. Three primer sequences with a 19bp transposase identification sequence, sequence A, sequence B and sequence C were designed and prepared, for preparation of NO. 1 adapter in the form of double-adapters (referred to herein as the NO. 1 adapter) for embedding, wherein, sequence A + sequence B forms the 5' end of the NO. 1 adapter in the form of double-adapters, and sequence A + sequence C forms the 3' end of the NO. 1 adapter in the form of double-adapters:
Sequence A of the NO. 1 adapter in the form of double-adapters:
   CTGTCTCTTATACACATCT (SEQ ID NO: 1);
Sequence B of the NO. 1 adapter in the form of double-adapters:
   TCGTCGGCAGCGTCAGATGTGTATAAGAGACAG (SEQ ID NO: 2);
Sequence C of the NO. 1 adapter in the form of double-adapters:
   GTCTCGTGGGCTCGGAGATGTGTATAAGAGACAG (SEQ ID NO: 3).

2. The sequence A, sequence B and sequence C were diluted to 100µM respectively, sequence A + sequence B combination, sequence A + sequence C combination, fully mixed and centrifuged, and then annealed to form NO. 1 adapter (stored at -20 °C) in a PCR instrument according to the following procedure (Table 1), and used for the preparation of embedded composites.

**Table 1**

| Temperature | Time |
|---|---|
| 75°C | 15min |
| 60°C | 10min |
| 50°C | 10min |
| 40°C | 10min |
| 25°C | 30min |
| Hot-lid 105°C | |

After the reaction, the two sets of annealed adapters were mixed in equal volume, for embedding the transposase complex.
3. The NO. 1 adapter and the transposase were embedded into a transposase-embedded complex according to the following system (Table 2), after gently blowing 20 times and incubating 1 hour at 30 °C, the complex embedding was completed. The complex was stored at -20°C.

**Table 2**

| Component | Content |
|---|---|
| Transposase S50 reagent | 47µL |
| NO. 1 adapter | 2µL |
| Coupling buffer | 47µL |
| Total | 96µL |

4. 50ng of high quality genome and transposase complex were mixed according to the following system (Table 3), after gently mixing 20 times and incubating for 10 minutes at 55°C, and then cooling to 4°C, genome interruption is completed.

**Table 3**

| Component | Content |
|---|---|
| Water | 5µL |
| 5× interruption buffer | 2µL |
| gDNA (50ng/µL) | 1µL |
| Transposase complex | 2µL |
| Total | 10µL |

5. The sample processing methods after the interruption comprises the following options. Method 1: adding 1 times of the volume of PBI (a commercial reagent in Qiagen PCR purification kit), after mixing evenly, purifying with 1.3 times of Ampure XP beads, and dissolving with pure water. Method 2: 0.1-5 µL of protease (750 mAU / mL) was added and then added to a final concentration of 1-50 mM EDTA. This example preferred 2 µL of protease and final concentration of 14 mM EDTA, and at the same time 0.1µL protease plus 1 mM EDTA and 5µL of protease plus 50 mM EDTA was tested. Method 3: adding 0.01% to 1.5% (by volume) of SDS, preferably 1% (by volume) of SDS in this example, and 0.01% (by volume) and 1.5% (by volume) concentrations were tested separately. Method 4: adding the final concentration of commercial 1 × NT buffer (a matching reagent in Truprep kit S5 series). Method 5: adding 0.1-5 µL of protease for treatment, preferably 2µL of protease in this example, and 0.1µL and 5µL protease were tested separately.
6. In the product after the above treatment, 0.1%-2% (by volume) of Triton-X100 was added, preferably 1% (by volume) in this example, while 0.1% (by volume) and 2% (by volume) of Triton-X100 was used to test.
7. PCR amplification was carried out according to the following PCR reaction system (Table 4) and reaction conditions (Table 5). For the experimental group with SDS added, a specific concentration of Tween-20 was added to the PCR system to partially increase the efficiency of the PCR. The working concentration of Tween-20 could be adjusted to different, such as 0.1% -2% (by volume), preferably 0.5% (by volume) in this example, while the working concentrations of 0.1% (by volume) and 2% (by volume) was tested.

**Table 4**

| Component | Content |
|---|---|
| Processed DNA samples | 30µL |
| 5× PCR buffer (containing Mg²⁺) | 10µL |
| 10mM dNTP | 1µL |
| Primer 1 (10µM) | 2µL |
| Primer 2 (10µM) | 2µL |
| PCR enzyme (DNA polymerase) | 1µL |
| Pure water | 4µL |
| Total | 50 µL |

| | |
|---|---|
| Note: Primer 1 of the NO. 1 adapter in the form of double-adapters: AATGATACGGCGACCACCGA (SEQ ID NO: 4); Primer 2 of the NO. 1 adapter in the form of double-adapters: CAAGCAGAAGACGGCATACGA (SEQ ID NO: 5). | |

**Table 5**

| Temperature | Time | Cycle |
|---|---|---|
| 72°C | 3min | 1 Cycle |
| 98°C | 30sec | 1 Cycle |
| 98°C | 10sec | 15 Cycles |
| 60°C | 30sec | |
| 72°C | 3min | |
| 72°C | 5min | 1 Cycle |
| 4°C | For ever | - |

8. PCR product detection result of the transposase complex of the NO. 1 adapter in the form of double-adapters is shown in Figure 2, and the PCR product concentration determination results are shown in Table 6:

**Table 6**

| Group | Processing method after interruption | PCR product concentration (ng/µL) | Remarks (Fig. 2) |
|---|---|---|---|
| 1 | 1×PBI, 1.3×Ampure XP beads | 22.8 | Lane 1 |
| 2 | 2µL protease+14mM EDTA+1% Triton-X100 | 20.2 | Lane 2 |
| 3 | 1% SDS +1% Triton-X100+0.5% Tween-20 | 22.4 | Lane 3 |
| 4 | NT buffer+1% Triton-X100 | 25 | Lane 4 |
| 5 | 2µL protease+1% Triton-X100 | 20 | Lane 5 |
| 6 | 0.1µL protease+1mM EDTA+0.1 % Triton-X 100 | 9 | - |
| 7 | 5µL protease+50mM EDTA+2% Triton-X100 | 18 | - |
| 8 | 0.01% SDS+0.1% Triton-X100+0.1% Tween-20 | 8.8 | - |
| 9 | 1.5% SDS+2% Triton-X100+2% Tween-20 | 17 | - |
| 10 | 0.1µL protease+0.1% Triton-X100 | 6 | - |
| 11 | 5µL protease+2% Triton-X100 | 18 | - |

### Example 2

In this example, 50 ng of high quality genomic DNA was first interrupted by an embedded transposase complex, treated with protease, SDS, NT or protease and EDTA compositions to remove the transposase protein bound to DNA. After the ligation of the gap adapter, PCR primers were used to amplify, and a certain concentration of TritonX-100 was added to the PCR reaction system.
1. A pair of primer sequences, sequences A and B, with a 19bp transposase identification sequence were designed and used to prepare a NO. 1 adapter in the form of single-adapter for embedding (referred to as the NO. 1 adapter in the present invention).
Sequence A of the NO. 1 adapter in the form of single-adapter:
   TCGTCGGCAGCGTCAGATGTGTATAAGAGACAG (SEQ ID NO: 6);
Sequence B of the NO. 1 adapter in the form of single-adapter:
   CTGTCTCTTATACACATC ddT (SEQ ID NO: 7, dd represents dideoxy modification).

2. The sequence A and sequence B were diluted to 100µM respectively, fully mixed and centrifuged, and then annealed to form NO. 1 adapter (stored at -20°C) in a PCR instrument according to the following procedure (Table 7), and used for the preparation of embedded composites.

**Table 7**

| Temperature | Time |
|---|---|
| 75°C | 15min |
| 60°C | 10min |
| 50°C | 10min |
| 40°C | 10min |
| 25°C | 30min |
| Hot-lid 105°C | |

3. The NO. 1 adapter and the transposase were embedded into a transposase-embedded complex according to the following system (Table 8), after gently blowing 20 times and incubating 1 hour at 30 °C, the complex embedding was completed. The complex was stored at -20°C.

**Table 8**

| Component | Content |
|---|---|
| Transposase | 85µL |
| NO. 1 adapter | 30µL |
| Coupling buffer | 85µL |
| Total | 200µL |

4. 50ng of high quality genome and transposase complex were mixed according to the following system (Table 9), after gently mixing 20 times and incubating for 10 minutes at 55°C, and then cooling to 4°C, genome interruption is completed.

**Table 9**

| Component | Content |
|---|---|
| Water | 5µL |
| 5× interruption buffer | 2µL |
| gDNA (50ng/µL) | 1µL |
| Transposase complex | 2µL |
| Total | 10µL |

5. The sample processing methods after the interruption comprises the following options. Method 1: 0.1-5 µL of protease (750 mAU / mL) was added, in this example preferred 2 µL of protease, and at the same time 0.1µL protease and 5µL of protease was tested respectively. Method 2: adding the final concentration of commercial 1 × NT buffer (a matching reagent in Truprep kit S5 series). Method 3: adding 0.01% to 1.5% (by volume) of SDS, preferably 1% (by volume) of SDS in this example, and 0.01% (by volume) and 1.5% (by volume) concentrations were tested separately. Method 4: 0.1-5 µL of protease (750 mAU / mL) was added and then added to a final concentration of 1-50 mM EDTA. This example preferred 2 µL of protease and final concentration of 14 mM EDTA, and at the same time 0.1µL protease plus 1 mM EDTA and 5µL of protease plus 50 mM EDTA was tested. Method 5: adding 1 times of the volume of PBI (a commercial reagent in Qiagen PCR purification kit), after mixing evenly, purifying with 1.3 times of Ampure XP beads, and dissolving with pure water.
6. In the product after the above treatment, 0.1%-2% (by volume) of Triton-X100 was added, preferably 1% (by volume) in this example, while 0.1% (by volume) and 2% (by volume) of Triton-X100 was used to test.
7. The product treated with the above methods was ligated to the annealed gap adapter (the NO. 2 adapter) according to the following system (Table 10), after annealing at 25°C for 60 minutes, the adapter ligation was completed.

**Table 10**

| Component | Content |
|---|---|
| Water | 8µL |
| 3×ligation buffer | 20µL |
| NO. 2 adapter (5µM) | 10µL |
| Ligase | 2µL |
| DNA | 20µL |
| Total | 30µL |

| | |
|---|---|
| Note: Sequence A of the NO. 2 adapter: 5'-pAAGTCGGAGGCCAAGCGGTCGT ddC-3' (SEQ ID NO: 8); Sequence B of the NO. 2 adapter: 5'-TTGGCCTCCGACT ddT-3' (SEQ ID NO: 9) (p represents phosphorylation modification , dd represents dideoxy modification). | |

8. PCR amplification was carried out according to the following PCR reaction system (Table 11) and reaction conditions (Table 12). For the experimental group with SDS added, a specific concentration of Tween-20 was added to the PCR system to partially increase the efficiency of the PCR. The working concentration of Tween-20 could be adjusted to different, such as 0.1% -2% (by volume), preferably 0.5% (by volume) in this example, while the working concentrations of 0.1% (by volume) and 2% (by volume) was tested.

**Table 11**

| Component | Content |
|---|---|
| Processed DNA samples | 30µL |
| 5× PCR buffer | 10µL |
| 10mM dNTP | 1µL |
| Primer 1 | 2µL |
| Primer 2 | 2µL |
| PCR enzyme (DNA polymerase) | 1µL |
| Pure water | 4µL |
| Total | 50µL |

| | |
|---|---|
| Note: Primer 1 of the NO. 1 adapter in the form of single-adapter: AGACAAGCTCGAGCTCGAGCGATCGGGATCTACACGACTCACTGATC GTCGGCAGCGTC (SEQ ID NO: 10); Primer 2 of the NO. 1 adapter in the form of single-adapter: TCCTAAGACCGCTTGGCCTCCGACT (SEQ ID NO: 11). | |

**Table 12**

| Temperature | Time | Cycle |
|---|---|---|
| 72°C | 3min | 1 Cycle |
| 98°C | 30sec | 1 Cycle |
| 98°C | 10sec | 15 Cycles |
| 60°C | 30sec | |
| 72°C | 3min | |
| 72°C | 5min | 1 Cycle |
| 4°C | For ever | - |

9. PCR product detection result of after interruption by single-adapter embedding complex and ligation of the gap adapter is shown in Figure 3, and the PCR product concentration determination results are shown in Table 13.

**Table 13**

| Group | Processing method after interruption | PCR product concentration (ng/µL) | Remarks (Fig. 3) |
|---|---|---|---|
| 1 | 2µL protease +1% Triton-X100 | 11.4 | Lane 1 |
| 2 | NT buffer+1% Triton-X100 | 13 | Lane 2 |
| 3 | 1% SDS +1% Triton-X100+0.5% Tween-20 | 12.4 | Lane 3 |
| 4 | 2µL protease +14mM EDTA+1% Triton-X100 | 12 | Lane 4 |
| 5 | 1×PBI, 1.3×Ampure XP beads +1%Triton-X100 | 13.5 | Lane 5 |
| 6 | 0.1µL protease+1mM EDTA+0.1% Triton-X100 | 6.2 | - |
| 7 | 5µL protease +50mM EDTA+2% Triton-X100 | 10.3 | - |
| 8 | 0.01% SDS+0.1% Triton-X100+0.1% Tween-20 | 5.3 | - |
| 9 | 1.5% SDS+2% Triton-X100+2% Tween-20 | 9.1 | - |
| 10 | 0.1µL protease +0.1% Triton-X100 | 6 | - |
| 11 | 5µL protease +2% Triton-X100 | 10.1 | - |

The foregoing is a further detailed description of the present invention in reference with the specific embodiments, thus it cannot be determined that the specific implementation of the invention is limited to these above illustrations. It will be apparent to one skilled in the art to which the invention pertains that several simple deductions or substitutions may be made without departing from the inventive concept as defined in the appended claims.

## Claims

1. A treatment method for breaking a nucleic acid by means of a transposase comprising the following steps:
randomly interrupting a nucleic acid using a transposase-embedded complex, wherein the transposase-embedded complex comprises a transposase and a first adapter comprising a transposase identification sequence;
adding a first reagent for treatment, so as to break the adsorption effect of the transposase and the target sequence of the nucleic acid;
adding a second reagent for treatment, so as to weaken the influence of the first reagent on the subsequent enzymatic reactions; and
performing a PCR reaction by using a product generated after the second reagent treatment as a template component, so as to obtain a PCR product with an adapter at each end of the interrupted nucleic acid fragment;
wherein the first reagent comprises a SDS solution;
wherein the second reagent comprises Triton-X100 solution, and further comprises a Tween-20 solution.

2. The method of claim 1 wherein after adding the second reagent for treatment and before performing the PCR reaction, a second adapter is ligated at a gap by a ligase, wherein the gap is a 9bp base deletion formed after both ends of the interrupted nucleic acid each ligated to the first adapter.

3. The method of claim 2 wherein the 3' end of the second adapter is a dideoxynucleotide to prevent the second adapter from ligating to the first adapter.

4. A treatment reagent set for breaking a nucleic acid by means of a transposase comprising the following components:
a first reagent comprising a SDS solution to break the adsorption effect of the transposase and the target sequence of the nucleic acid; and
a second reagent comprising a Triton-X100 solution and a Tween-20 solution to weaken the influence of the first reagent on the subsequent enzymatic reactions.

5. The reagent of claim 4 wherein the reagent further comprises:
a transposase and a first adapter comprising a transposase identification sequence, for forming a transposase-embedded complex to randomly interrupte the nucleic acid;
preferably, the reagent further comprises PCR components for carrying out a PCR reaction using the product generated by the treatment of the second reagent as a template component;
preferably, the reagent further comprises a second adapter component for ligating into the gap formed by ligating the first adapter to the interrupted nucleic acid at both ends;
preferably, the reagent further comprises a ligase component for ligating a second adapter to the gap formed by ligating the first adapter to the interrupted nucleic acid at both ends.

## Patentansprüche

1. Behandlungsverfahren zum Brechen einer Nukleinsäure mittels einer Transposase, umfassend die folgenden Schritte:
willkürliches Unterbrechen einer Nukleinsäure mit einem Transposase-eingebetteten Komplex, wobei der Transposaseeingebettete Komplex eine Transposase und einen ersten Adapter, umfassend eine Transposase-Erkennungssequenz, umfasst;
Zufügen eines ersten Reagenzes zur Behandlung, um auf diese Weise die Adsorptionswirkung der Transposase und der Zielsequenz der Nukleinsäure zu brechen;
Zufügen eines zweiten Reagenzes zur Behandlung, um auf diese Weise den Einfluss des ersten Reagenzes auf die sich anschließenden enzymatischen Reaktionen zu dämpfen; und
Durchführen einer PCR-Reaktion mittels eines Produkts, erzeugt nach der Behandlung des zweiten Reagenzes als eine Matrizenkomponente, um auf diese Weise ein PCR-Produkt mit einem Adapter an jedem Ende des unterbrochenen Nukleinsäurefragments zu erhalten;
wobei das erste Reagenz eine SDS-Lösung umfasst;
wobei das zweite Reagenz eine Triton X-100-Lösung umfasst, und ferner eine Tween 20-Lösung umfasst.

2. Verfahren nach Anspruch 1, wobei nach dem Zufügen des zweiten Reagenzes zur Behandlung und vor dem Durchführen der PCR-Reaktion, ein zweiter Adapter mittels einer Ligase an eine Lücke (Gap) ligiert wird, wobei die Lücke (Gap) eine Basen-Deletion von 9 bp ist, gebildet nachdem beide Enden der unterbrochenen Nukleinsäure jeweils an den ersten Adapter ligiert sind.

3. Verfahren nach Anspruch 2, wobei das 3'-Ende des zweiten Adapters ein Didesoxynukleotid ist, um den zweiten Adapter am Ligieren an den ersten Adapter zu hindern.

4. Behandlungsreagenzset zum Brechen einer Nukleinsäure mittels einer Transposase, umfassend die folgenden Komponenten:
ein erstes Reagenz, umfassend eine SDS-Lösung zum Brechen der Adsorptionswirkung der Transposase und der Zielsequenz der Nukleinsäure; und
ein zweites Reagenz, umfassend eine Triton X-100-Lösung und eine Tween 20-Lösung zum Dämpfen des Einflusses des ersten Reagenzes auf die sich anschließenden enzymatischen Reaktionen.

5. Reagenz nach Anspruch 4, wobei das Reagenz ferner Folgendes umfasst:
eine Transposase und einen ersten Adapter, umfassend eine Transposase-Erkennungssequenz, zum Bilden eines Transposase-eingebetteten Komplexes zum willkürlichen Unterbrechen der Nukleinsäure;
das Reagenz bevorzugt ferner PCR-Komponenten zum Durchführen einer PCR-Reaktion mit dem Produkt, erzeugt durch die Behandlung des zweiten Reagenzes als eine Matrizenkomponente, umfasst;
das Reagenz bevorzugt ferner eine zweite Adapter-Komponente zum Ligieren in die Lücke (Gap), gebildet mittels Ligieren des ersten Adapters an die unterbrochene Nukleinsäure an beiden Enden, umfasst;
das Reagenz bevorzugt ferner eine Ligase-Komponente zum Ligieren eines zweiten Adapters an die Lücke (Gap), gebildet mittels Ligieren des ersten Adapters an die unterbrochene Nukleinsäure an beiden Enden, umfasst.

## Revendications

1. Procédé de traitement pour rompre un acide nucléique au moyen d'une transposase comprenant les étapes suivantes :
l'interruption aléatoire d'un acide nucléique à l'aide d'un complexe intégré dans une transposase, où le complexe intégré dans une transposase comprend une transposase et un premier adaptateur comprenant une séquence d'identification de transposase ;
l'ajout d'un premier réactif de traitement, de manière à rompre l'effet d'adsorption de la transposase et de la séquence cible de l'acide nucléique ;
l'ajout d'un deuxième réactif de traitement, de manière à affaiblir l'influence du premier réactif sur les réactions enzymatiques ultérieures ; et
la réalisation d'une réaction PCR par utilisation d'un produit généré après le traitement par le deuxième réactif comme composant modèle, de manière à obtenir un produit PCR avec un adaptateur à chaque extrémité du fragment d'acide nucléique interrompu ;
où le premier réactif comprend une solution de SDS ;
où le deuxième réactif comprend une solution de Triton-X100, et comprend en outre une solution de Tween-20.

2. Procédé selon la revendication 1 où, après l'ajout du deuxième réactif pour le traitement et avant la réalisation de la réaction PCR, un deuxième adaptateur est ligaturé au niveau d'un intervalle par une ligase, où l'intervalle est une délétion de base de 9 pb formée après la ligature de chacune des deux extrémités de l'acide nucléique interrompu au premier adaptateur.

3. Procédé selon la revendication 2 où l'extrémité 3' du deuxième adaptateur est un didésoxynucléotide pour empêcher la ligature du deuxième adaptateur au premier adaptateur.

4. Ensemble de réactifs de traitement pour rompre un acide nucléique au moyen d'une transposase comprenant les composants suivants :
un premier réactif comprenant une solution de SDS pour rompre l'effet d'adsorption de la transposase et de la séquence cible de l'acide nucléique ; et
un deuxième réactif comprenant une solution de Triton-X100 et une solution de Tween-20 pour affaiblir l'influence du premier réactif sur les réactions enzymatiques ultérieures.

5. Réactif selon la revendication 4 où le réactif comprend en outre :
une transposase et un premier adaptateur comprenant une séquence d'identification de transposase, afin de former un complexe intégré dans une transposase pour interrompre aléatoirement l'acide nucléique ;
de préférence, le réactif comprend en outre des composants PCR afin d'effectuer une réaction PCR à l'aide du produit généré par le traitement du deuxième réactif comme composant modèle ;
de préférence, le réactif comprend en outre un deuxième composant adaptateur de ligature dans l'intervalle formé par ligature du premier adaptateur à l'acide nucléique interrompu aux deux extrémités ;
de préférence, le réactif comprend en outre un composant ligase pour la ligature d'un deuxième adaptateur à l'intervalle formé par ligature du premier adaptateur à l'acide nucléique interrompu aux deux extrémités.


### SEQUENCE LISTING

<110> BGI SHENZHEN CO., LIMITED
<120> ONE-STOP TREATMENT METHOD FOR BREAKING NUCLEIC ACID BY MEANS OF
   TRANSPOSASE, AND REAGENT
<130> 14P20608
<160> 11
<170> PatentIn version 3.3
<210> 1
   <211> 19
   <212> DNA
   <213> Artificial sequence
<400> 1
   ctgtctctta tacacatct 19
<210> 2
   <211> 33
   <212> DNA
   <213> Artificial sequence
<400> 2
   tcgtcggcag cgtcagatgt gtataagaga cag 33
<210> 3
   <211> 34
   <212> DNA
   <213> Artificial sequence
<400> 3
   gtctcgtggg ctcggagatg tgtataagag acag 34
<210> 4
   <211> 20
   <212> DNA
   <213> Artificial sequence
<400> 4
   aatgatacgg cgaccaccga 20
<210> 5
   <211> 21
   <212> DNA
   <213> Artificial sequence
<400> 5
   caagcagaag acggcatacg a 21
<210> 6
   <211> 33
   <212> DNA
   <213> Artificial sequence
<400> 6
   tcgtcggcag cgtcagatgt gtataagaga cag 33
<210> 7
   <211> 19
   <212> DNA
   <213> Artificial sequence
<220>
   <221> dideoxy modification
   <222> (19)..(19)
<400> 7
   ctgtctctta tacacatct 19
<210> 8
   <211> 23
   <212> DNA
   <213> Artificial sequence
<220>
   <221> phosphorylation modification
   <222> (1)..(1)
<220>
   <221> dideoxy modification
   <222> (23)..(23)
<400> 8
   aagtcggagg ccaagcggtc gtc 23
<210> 9
   <211> 14
   <212> DNA
   <213> Artificial sequence
<220>
   <221> dideoxy modification
   <222> (14)..(14)
<400> 9
   ttggcctccg actt 14
<210> 10
   <211> 59
   <212> DNA
   <213> Artificial sequence
<400> 10
   agacaagctc gagctcgagc gatcgggatc tacacgactc actgatcgtc ggcagcgtc 59
<210> 11
   <211> 25
   <212> DNA
   <213> Artificial sequence
<400> 11
   tcctaagacc gcttggcctc cgact 25